(19) Europäisches Patentamt
European Patent Office
Office européen des brevets

(11) **EP 3 995 567 A1**

(12) **EUROPEAN PATENT APPLICATION**
published in accordance with Art. 153(4) EPC

(43) Date of publication:
**11.05.2022 Bulletin 2022/19**

(21) Application number: **20835360.7**

(22) Date of filing: **06.07.2020**

(51) International Patent Classification (IPC):
***C12N 1/14*** (2006.01) ***C12N 9/24*** (2006.01)
***C12P 19/14*** (2006.01) ***C12P 19/00*** (2006.01)
***C12P 19/12*** (2006.01) ***C12P 19/04*** (2006.01)

(52) Cooperative Patent Classification (CPC):
**C12N 1/14; C12N 9/24; C12P 19/00; C12P 19/04; C12P 19/12; C12P 19/14**

(86) International application number:
**PCT/CN2020/100482**

(87) International publication number:
**WO 2021/000963 (07.01.2021 Gazette 2021/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.07.2019 CN 201910596633**
**04.07.2019 CN 201910597371**
**19.10.2019 CN 201910996538**
**19.10.2019 CN 201910997521**

(71) Applicant: **Shandong Bailong Chuangyuan Bio-tech Co., Ltd**
**Dezhou, Shandong 251200 (CN)**

(72) Inventors:
- **GAN, Zhaobo**
**DEZHOU (YUCHENG), Shandong 251200 (CN)**
- **DOU, Baode**
**DEZHOU (YUCHENG), Shandong 251200 (CN)**
- **DOU, Guangpeng**
**DEZHOU (YUCHENG), Shandong 251200 (CN)**
- **SHAO, Xianbao**
**DEZHOU (YUCHENG), Shandong 251200 (CN)**
- **LI, Fanghua**
**DEZHOU (YUCHENG), Shandong 251200 (CN)**
- **DU, Qian**
**DEZHOU (YUCHENG), Shandong 251200 (CN)**

(74) Representative: **Held, Stephan**
**Meissner Bolte Patentanwälte**
**Rechtsanwälte Partnerschaft mbB**
**Widenmayerstraße 47**
**80538 München (DE)**

(54) **TRICHODERMA REESEI STRAIN, CULTURE METHOD THEREFOR, AND APPLICATION THEREOF**

(57) The invention relates to a strain of *Trichoderma reesei* BLCY-007 and its application in the production of xylooligosaccharides.

EP 3 995 567 A1

## Description

This application is based on CN patent application 201910597371.5 with application date of July 4, 2019; CN patent application 201910596633.6 with application date of July 4, 2019; CN patent application 201910996538.5 with application date of October 19, 2019 Date; and CN patent application 201910997521.1 with application date of October 19, 2019, and claims the priority of them, and the disclosures of the above CN applications are hereby incorporated into the present application in their entirety.

## Technical Field

The invention relates to a strain of *Trichoderma reesei* and its culture method and use thereof, belonging to the technical field of microorganisms.

## Background Art

With the rapid development of economy and society, understanding of the nutrition and function of food is gradually improved, and more attention is paid to improve the health condition by improving dietary conditions and exerting the physiological regulation function of food itself. Oligosaccharides, also known as oligoses, are the general term for linear or branched low-degree polysaccharides formed by connecting 2 to 10 monosaccharides via glycosidic bonds, and they have a molecular weight of about 300 to 2000. Oligosaccharides have special biological functions, in particular, they can promote the proliferation of bifidobacteria in the intestine and are beneficial to human intestinal health. Among them, the most effective are xylooligosaccharides, which have an efficacy of nearly 20 times that of other polysaccharides. There is no enzyme in the human gastrointestinal tract that can hydrolyze xylooligosaccharides, and xylooligosaccharides can directly enter the large intestine and be preferentially utilized by bifidobacteria, which will promote the proliferation of bifidobacteria, and meantime produce a variety of organic acids, reduce intestinal pH and inhibit the growth of harmful bacteria, promote the proliferation of probiotics in the intestinal tract, and achieve beneficial effects on health.

Xylooligosaccharides are a mixture of oligosaccharides which contain 2 to 9 xylose units connected by β-1,4 glycosidic bonds, and their structural formula is as follows, n = 2 to 9,

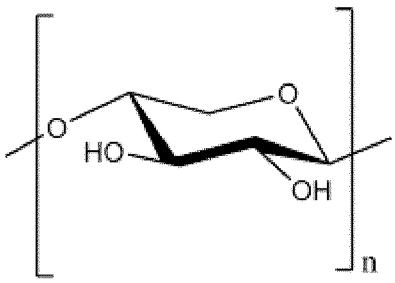

Xylooligosaccharides are a kind of oligosaccharide made from natural dietary fibers such as corncobs, cottonseed hulls, bagasse and other natural food fibers by saccharification and decomposition of hemicellulose with xylanase.

Chinese patent application CN105154412A discloses a method for extracting xylanase from bags of waste snow fungus, which belongs to the field of biological fermentation engineering. In this method, bags of waste snow fungus were added with water for extraction, the extract liquid was subjected to salting out with ammonium sulfate, then salt was removed by dialyzing, and then the resultant was purified by DEAE-cellulose column chromatography to obtain xylanase. However, the method in this patent is complicated in xylanase extraction, separation and purification processes, and is not well suitable for large-scale industrial production.

In addition, it is also an efficient way to produce xylanase by using microorganisms. For example: *Penicillium occitanis* Pol6 and *Aspergillus niger* BCC14405 show significant yield advantages in the production of xylanase, but the production of xylan with them are often accompanied by the production of toxins, therefore, such xylanase has certain hidden dangers in applications.

Some Trichoderma can also be used to produce xylanase. For example, patent document IN201741043810A discloses a new Trichoderma strain GAMSII M501, whose deposit number is MTCC25104. The strain can be used to produce a highly active enzyme mixture containing cellulase and xylanase. The production method thereof includes the following steps: a) culturing the cells of the new natural strain of *T.gamsii* M501 having an accession number of MTCC25104 in a modified Vogel's medium, which is supplemented with 1% microcrystalline cellulose and whose pH is

adjusted to 5.5, b) culturing the cells at a temperature of about 28°C for 3 days to obtain a culture, c) obtaining a culture supernatant from the culture containing cellulase and xylanase. The maximum levels of filter paper enzyme activity FPase, carboxymethyl cellulase enzyme activity CMCase and xylanase enzyme activity produced by the new strain of *T.gamsii* were 2.0 U/ml, 45.3 U/ml and 600 U/ml, respectively. The resulting enzyme mixture can be used for the hydrolysis of alkali-pretreated lignocellulosic biomass. However, the enzyme obtained from this strain is a mixture, and cannot be directly applied to the production of xylooligosaccharides.

## Contents of the Invention

Aiming at the deficiencies of the prior art, the present invention provides a *Trichoderma reesei* strain as well as culture method and use thereof. The crude enzyme preparation obtained based on the *Trichoderma reesei* has a high xylanase activity and does not contain other unwanted enzymes, and can be directly applied to produce xylooligosaccharides.

The technical solution of the present invention is as follows:

A strain of *Trichoderma reesei* BLCY-007, which has an Accession number: CGMCC No. 17970; the number was obtained when the strain was deposited on June 14, 2019 in China General Microbiological Culture Collection Center (CGMCC), the Institute of Microbiology, Chinese Academy of Sciences, at the address: No.1, West Beichen Road, Chaoyang District, Beijing.

*Trichoderma reesei* is a fungus of the genus *Trichoderma. Trichoderma* fungi belong to family *Gloiosporae,* order *Hyphomycetales,* class *Hyphomycetes,* subphylum *Deuteromycotina.*

The original strain of the novel *Trichoderma reesei* BLCY-007 of the present invention was isolated from the soil near the Research and Development pilot plant of Bailong Chuangyuan, Dezhou City, Shandong Province. After the original strain was mutagenized by ultraviolet radiation and mutagen treatment, the novel strain was obtained and named as *Trichoderma reesei* BLCY-007.

The fungal colony of this strain has a spread floccule like morphology. At first, it has a white dense flat hyphae, and then a light green sporulation cluster appears on its back edge, and the reverse side is colorless. The short side branches of conidiophore hyphae are transparent, multi-branched; the sterigma is bottle-shaped, curved in the middle; the conidia are in oval or long shape, single cell, transparent, colorless, with smooth wall, having a color of green when being piled.

A crude enzyme preparation can be obtained by simple centrifugation/filtration of a fungal fermentation broth of the strain. The crude enzyme preparation can hydrolyze xylan, and the xylanase enzyme activity of the crude enzyme preparation can reach 508U/ml in the optimum pH range of 5.5 to 6.5.

A culture method (or fermentation method) for the aforementioned *Trichoderma reesei* BLCY-007 comprises the following steps:

(i) inoculating *Trichoderma reesei* BLCY-007 into a PDA medium, and performing an activating cultivation under a temperature from 24°C to 28°C for 12 to 24 hours to obtain an activated strain;

(ii) inoculating the activated strain obtained in step (i) into a seed culture medium, and performing a proliferating cultivation under a temperature from 24°C to 28°C for 24 to 36 hours to prepare a seed broth;

(iii) inoculating the seed broth prepared in step (ii) into a fermentation medium at a volume percentage of 1% to 10%, and performing an expanding cultivation at a temperature from 24°C to 28°C for 24 to 36 hours to obtain a fungal fermentation broth.

In some embodiments, the method for preparing the fungal fermentation broth of *Trichoderma reesei* BLCY-007 comprises:

(i) inoculating the *Trichoderma reesei* BLCY-007 into a PDA medium, performing an activating cultivation under a temperature from 24°C to 28°C for 12 to 24 hours to obtain an activated strain;

(ii) inoculating the activated strain obtained in step (i) into a seed culture medium, and performing a proliferating cultivation under a temperature from 24°C to 28°C for 24 to 36 hours to prepare a seed broth;

(iii) inoculating the seed broth prepared in step (ii) into a fermentation medium at a volume percentage of 1% to 10%, and performing an expanding cultivation at a temperature from 24°C to 28°C for 24 to 36 hours to obtain a fungal fermentation broth.

In some embodiments, the seed medium in step (ii) has raw material components as follows:
200g of peeled potato, 20g of glucose, 3g of $KH_2PO_4$, 1.5g of $MgSO_4 \cdot 7H_2O$. After the above components are mixed, 1.0L of water is added and boiled for 30min, filtered to remove potato pieces, and the filtrate is supplemented to 1.0L.

In some embodiments, the seed medium in step (ii) has raw material components as follows:
as expressed in weight parts, 150 to 250 parts of peeled potato, 15 to 25 parts of glucose, 2 to 4 parts of $KH_2PO_4$, 1 to 2 parts of $MgSO_4 \cdot 7H_2O$, the above components are mixed, 1000 parts of water are added and boiled for 30min, filtered to remove potato pieces, and the filtrate is supplemented to 1000 parts.

In some embodiments, the fermentation medium in step (iii) has raw material components as follows, in terms of percentages by weight:
corncobs 25%, glucose 4%, beef extract 6%, peptone 1%, anhydrous magnesium sulfate 0.01%, dipotassium hydrogen phosphate 0.02%, ammonium sulfate 0.02%, balance of water, pH = 5.0 to 6.0.

In some embodiments, the fermentation medium in step (iii) has raw material components as follows, in terms of percentages by weight:
corncobs 20% to 30%, glucose 3% to 5%, beef extract 5% to 7%, peptone 0.5% to 2%, anhydrous magnesium sulfate 0.01% to 0.02%, dipotassium hydrogen phosphate 0.01% to 0.03%, ammonium sulfate 0.01% to 0.03 %, balance of water, pH = 5.0 to 6.0.

In some embodiments, the PDA medium in step (i) has raw material components as follows:
1.0L of potato extract liquid, 20.0g of glucose, 15.0g of agar.

The potato extract liquid is prepared by the following method, comprising: 200 g of peeled potato is taken, cut into small pieces, added with 1.0L of water and boiled for 30min, filtered to remove potato pieces, and the filtrate is supplemented to 1.0L.

In some embodiments, the PDA medium in step (i) has raw material components as follows:
expressed in weight parts, 80 to 120 parts of potato extract liquid, 25 to 25 parts of glucose, and 10 to 20 parts of agar.

Each 1000 parts of the potato extract liquid is prepared by the following method, comprising: 200 parts of peeled potato are taken, cut into small pieces, added with 1000 parts of water and boiled for 20 to 40 min, filtered to remove potato pieces, and the filtrate is supplemented to 1000 parts.

In some aspects, the present disclosure provides use of the aforementioned *Trichoderma reesei* BLCY-007 in the production of a xylanase.

In some embodiments, the xylanase refers to an enzyme preparation with a xylanase enzyme activity.

In some embodiments, the above use comprises steps as follows: the fungal fermentation broth prepared above is taken and subjected to centrifugation separation, and a supernatant is collected as the crude enzyme preparation.

In some embodiments, the aforementioned centrifugation separation is performed under a condition of 4°C and 10000 r/min for 10 min.

In some embodiments, the use comprises steps as follows: the fungal fermentation broth prepared above is taken and subjected to centrifugation separation, the fungal cells are washed and subjected to second centrifugation, and the precipitate is retained as the crude xylanase enzyme preparation. In some embodiments, the fungal cells are washed using Tris-HCl buffer with pH=8.0 and a concentration of 50 mmol/L, and then subjected to centrifugation separation, the precipitate is retained to obtain the crude enzyme preparation.

In some embodiments, the centrifugation separation is performed under a condition of 4°C and 10000 r/min for 10 min.

In some embodiments, the present disclosure provides use of the xylanase (such as the crude xylanase enzyme preparation) prepared above in the production of xylooligosaccharides.

In some embodiments, the present disclosure provides use of *Trichoderma reesei* BLCY-007 prepared above in the production of xylooligosaccharides.

In some aspects, the present disclosure provides a method for preparing a crude enzyme preparation, comprising:

(1) providing the fungal fermentation broth obtained by the aforementioned culture method;

(2) subjecting the fungal fermentation broth to centrifugation separation;

(3) collecting a supernatant from the product of the previous step, wherein the supernatant is a crude xylanase enzyme preparation.

In some aspects, the present disclosure provides a method for preparing xylooligosaccharides, comprising:

(1) preparing a crude xylanase enzyme preparation according to any one of the methods of the present disclosure;

(2) subjecting a xylan to enzymolysis treatment using the crude xylanase enzyme preparation to obtain xylanoligosaccharides.

In some embodiments, the enzymolysis treatment is performed at a temperature from 50°C to 60°C.

In some embodiments, the enzymolysis treatment is performed at a pH of 5.5 to 6.5.

The present invention also provides a method for preparing xylooligosaccharides by high-temperature and high-pressure treatment. In the method, corncobs are subjected to a high-temperature and high-pressure treatment to obtain xylan, and then a xylanase is added to perform enzymolysis to obtain a crude xylan oligosaccharide solution. None of acid and alkali is used in the process, thereby avoiding the discharge of a large amount of sewage and easing the pressure of environmental protection. Meanwhile, only adding xylanase once is needed for the enzymolysis in the process, thereby reducing the production cost. In the present invention, the xylanase produced by the newly developed *Trichoderma reesei* is used for enzymolysis, achieving an enzyme activity as high as 508U/ml. This further improves the extraction efficiency of xylooligosaccharides.

In some aspects, a method for preparing xylooligosaccharides by high-temperature and high-pressure treatment is provided, which comprises the following steps:

preparing a premix by crushing and sieving corncobs and adding water thereto;

subjecting the premix to high-temperature and high-pressure treatment to obtain a crude extract liquid of xylan, wherein the treatment is performed at a treatment temperature from 95°C to 140°C and a treatment pressure of 0.05 to 0.25 MPa;

adjusting the crude extract liquid of xylan to a mass concentration of 4% to 6% and a pH between 4.2 and 4.8, and performing a microwave treatment to obtain a xylan solution, wherein the microwave treatment is performed at a microwave frequency of 2450 MHz, a treatment temperature from 40°C to 55°C for a microwaving time of 10 to 25 minutes;

adding a xylanase into the xylan solution for enzymolysis to obtain a crude xylooligosaccharide solution;

subjecting the crude xylooligosaccharide solution to enzyme inactivation treatment, decolorization treatment, ion exchange treatment, and concentration treatment to obtain an xylooligosaccharide solution.

In some aspects, a method for preparing xylooligosaccharides by high-temperature and high-pressure treatment is provided, which comprises the following steps:

preparing a premix by crushing and sieving corncobs and adding water thereto;

subjecting the premix to high-temperature and high-pressure treatment to obtain a crude extract liquid of xylan, wherein the treatment is performed at a treatment temperature from 95°C to 140°C and a treatment pressure of 0.05 to 0.25 MPa;

adjusting the crude extract liquid of xylan to a mass concentration of 4% to 6% and a pH between 4.2 and 4.8, and performing a microwave treatment to obtain a xylan solution, wherein the treatment is performed at a treatment temperature from 40°C to 55°C for a microwaving time of 10 to 25 minutes;

adding a xylanase into the xylan solution for enzymolysis to obtain a crude xylooligosaccharide solution;

subjecting the crude xylooligosaccharide solution to enzyme inactivation treatment, decolorization treatment, ion exchange treatment, and concentration treatment to obtain an xylooligosaccharide solution.

In some aspects, a method for preparing xylooligosaccharides by high-temperature and high-pressure treatment is provided, which comprises the following steps:

(1) preparing a premix by crushing and sieving corncobs and adding water thereto;

(2) subjecting the premix to high-temperature and high-pressure treatment to obtain a crude extract liquid of xylan, wherein the treatment is performed at a treatment temperature from 95°C to 140°C and a treatment pressure of 0.05 to 0.25 MPa;

(3) adjusting the crude extract liquid of xylan to a dry matter mass concentration of 4% to 6% and a pH between 4.2 and 4.8, and performing a microwave treatment to obtain a xylan solution, wherein the treatment is performed at a microwave frequency of 2450 MHz, a microwave treatment temperature from 40°C to 55°C for a microwaving time of 10 to 25 minutes;

(4) adding a xylanase into the xylan solution for enzymolysis to obtain a crude xylooligosaccharide solution;

(5) subjecting the crude xylooligosaccharide solution to enzyme inactivation treatment, then decolorization treatment, ion exchange treatment, and concentration treatment to obtain an xylooligosaccharide solution.

In some embodiments, the corncobs are crushed to a particle size that is capable of passing through 80 to 120 mesh sieve, and the premix has a mass concentration of 8% to 12%.

In some embodiments, the high-temperature and high-pressure treatment is performed at a treatment temperature from 115°C to 128°C and a treatment pressure of 0.09 to 0.18 MPa for a treatment time of 4 to 8 hours.

In some embodiments, the xylan solution is adjusted to a mass concentration of 4% to 6% before enzymolysis; preferably, the xylanase is added in an amount of 4 to 6 g/kg dry matter; preferably, the enzymolysis is performed at a enzymolysis temperature from 50°C to 60°C for an enzymolysis time of 20 to 40 hours, and the enzymolysis is a static reaction.

In some embodiments, when adding a xylanase into the xylan solution for enzymolysis, the xylanase used is a xylanase produced by the following strain, and has an enzyme activity of 508 U/ml;
the strain is *Trichoderma reesei* BLCY-007, which has an Accession number: CGMCC No. 17970, which was obtained when the strain was deposited on June 14, 2019 in China General Microbiological Culture Collection Center, the Institute of Microbiology, Chinese Academy of Sciences, at the address: No.1, West Beichen Road, Chaoyang District, Beijing.

In some embodiments, the method for preparing xylooligosaccharides by high-temperature and high-pressure treatment refers to a method for preparing xylooligosaccharides by high-temperature and high-pressure treatment of corncobs.

In some embodiments, the premix of the step (1) does not contain acid or alkali.

In some embodiments, in step (1), the corncobs have a water content of 14% to 25%.

In some embodiments, the premix of the step (1) has a pH of 7 to 8.

In some embodiments, the premix in step (1) has a dry matter mass concentration of 8% to 12%.

In some embodiments, the dry matter mass concentration of the premix is calculated by the following formula: dry matter mass of corncobs /total mass of the premix× 100%.

In some embodiments, the dry matter refers to dry matter of corncobs, and the dry matter weight is a dry matter weight of corncobs.

In some embodiments, the dry matter mass concentration refers to a mass concentration based on the dry matter of corncobs.

In some embodiments, the step (2) is performed in a closed pressure container.

In some embodiments, the high temperature refers to a treatment temperature from 95°C to 140°C.

In some embodiments, the high pressure refers to a treatment pressure of 0.05 to 0.25 MPa.

In some embodiments, in step (2), the treatment temperature is 121°C and the treatment pressure is 0.1Mpa.

In some embodiments, the mass concentration in step (3) refers to a dry matter mass concentration of the crude extract liquid of xylan.

In some embodiments, in step (3), the microwave treatment refers to that the crude extract liquid of xylan is placed in a closed container, and then the container is placed in a microwave treatment equipment and subjected to microwave treatment.

In some embodiments, in step (3), the dry matter mass concentration of the crude extract liquid of xylan is adjusted to 4% to 6%, and the pH is adjusted to between 4.2 and 4.8 to obtain a pre-reaction solution, and the ratio of microwave power used in the microwave treatment to the pre-reaction solution is 500 to 1000 : 80 to 120 (W/ml), for example 700 to 900 : 100 to 110 (W/ml).

In some embodiments, in step (3), the crude extract liquid of xylan has a temperature from 40°C to 55°C during the microwave treatment.

In some embodiments, during the microwave treatment, the crude extract liquid of xylan has a temperature maintained at 40°C to 55°C.

In some embodiments, in step (4), the dry matter mass concentration of the xylan solution is adjusted to 4% to 6% before enzymolysis. The dry matter mass is calculated based on the dry matter of corncobs.

In some embodiments, in step (4), the enzymolysis is performed at an enzymolysis temperature from 50°C to 60°C for an enzymolysis time of 20 to 40 hours, and the xylan solution is kept static during the enzymolysis treatment.

In some embodiments, in step (4), only the crude enzyme preparation obtained from the fermentation broth of

*Trichoderma reesei* BLCY-007 strain is used as the xylanase.

The treatment temperature range of 95°C to 140°C and the pressure range of 0.05 to 0.25 MPa in step (2) are critical for increasing the yield of xylooligosaccharides. The xylan in the corncobs are a biological macromolecule, which in its natural state exists as a complex with other components such as cellulose and lignin, and the three have a discontinuous laminate structure, which hinders the hydrolysis. If the treatment is performed beyond the aforementioned temperature and pressure ranges, the discontinuous laminate structure cannot be destroyed, and the yield of xylan and the extraction rate of xylooligosaccharides will not be high.

It is found in the experiments that when the treatment pressure of the step (1) is lower than 0.05 MPa, the discontinuous laminate structure cannot be destroyed, and the yield of xylan and the extraction rate of xylooligosaccharides are not high. When the treatment pressure in step (1) is higher than 0.25MPa, xylan would be overly hydrolyzed to produce xylose, thereby reducing the yield of xylan and the extraction rate of xylooligosaccharides.

The crude enzyme preparation obtained from the fermentation broth of *Trichoderma reesei* BLCY-007 used in step (4) is also critical for increasing the yield of xylooligosaccharides.

In some embodiments, xylooligosaccharides are a mixture of oligosaccharides containing 2 to 9 xylose units connected by β-1,4 glycosidic bonds, and its structural formula is as follows, n = 2 to 9,

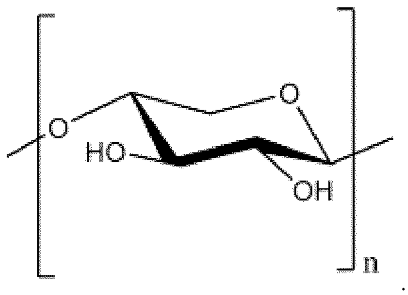

In some embodiments, the xylooligosaccharides meet the Chinese standard "GB/T 35545-2017 xylooligosaccharides".

In some embodiments, the xylan refers to a heterogeneous polysaccharide which exists in plant cell walls, accounting for about 15% to 35% of the dry weight of plant cells, and it is the main component of plant hemicellulose.

In some embodiments, the enzyme inactivation treatment method is performed by high temperature inactivation, at an enzyme inactivation temperature from 85°C to 98°C for an enzyme inactivation time of 10 to 15 minutes.

In some embodiments, the decolorization treatment is performed by using activated carbon to decolorize, the activated carbon is added in an amount of 0.8% to 5% of the dry matter mass in the crude xylooligosaccharide solution; the decolorization is performed at a temperature from 78°C to 85°C, at a liquid flow rate of 20 to 30 mL/min for a time of 15 to 30 minutes. The dry matter mass is calculated based on the dry matter weight of corncobs.

In some embodiments, the decolorization treatment is performed by using activated carbon to decolorize, the activated carbon is added in an amount of 0.8% to 5% of the dry matter mass in the crude xylooligosaccharide solution, the decolorization is performed at a temperature from 78°C to 85°C for a time of 15 to 30 minutes.

In some embodiments, the decolorization treatment refers to passing the crude xylooligosaccharide solution through an activated carbon filter element.

In some embodiments, the flow rate of the crude xylooligosaccharide solution through the filter element is 20 to 30 mL/min.

Preferably, in the ion exchange treatment process an ion exchange column that is a combined column of cation exchange column-anion exchange column-cation exchange column, is used, the ion exchange treatment temperature is 25°C to 35°C, and the ion exchange treatment is performed at a flow rate of 15 to 25 mL/min; further preferably, in the cation exchange column a strong acid cation resin is used, and in the anion exchange column a weak base anion resin is used.

In some embodiments, the strong acid cation resin is D001 macroporous cation exchange resin produced by Zhejiang Zhengguang Industrial Co., Ltd.

In some embodiments, the cation exchange resin is a styrene-divinylbenzene copolymer with sulfonic acid groups ($-SO_3H$).

In some embodiments, the weak base anion resin is D354FD macroporous weak base anion resin produced by Zhejiang Zhengguang Industrial Co., Ltd.

In some embodiments, the weak base anion resin is a weak base anion exchange resin with a polystyrene macroporous structure.

Preferably, the concentration treatment method is performed by rotary concentration under vacuum at a working

pressure of -0.1MPa, a working temperature from 60°C to 80°C, and the concentration of dry matter in the crude xylooligosaccharide solution is 60% to 78% after the concentration treatment.

According to a preferred embodiment of the present invention, the method for preparing xylooligosaccharides by high-temperature and high-pressure treatment comprises the following steps:

(1) pulping: a certain amount of corncobs are weighed, crushed and sieved with an 80 to 120 mesh sieve, then the resultant is pulped into a premix having a mass concentration of 8% to 12%, wherein water used for the pulping is pure water;

(2) high-temperature and high-pressure treatment: the premix in step (1) is subjected to high-temperature and high-pressure treatment to obtain a crude extract liquid of xylan, wherein the treatment is performed at a treatment temperature from 115°C to 128°C and a treatment pressure of 0.09 to 0.18 MPa for a treatment time of 4 to 8 hours,;

(3) microwave treatment: the crude extract liquid of xylan in step (2) is adjusted to a mass concentration of 4% to 6% and a pH of 4.2 to 4.8 to obtain a pre-reaction solution; the pre-reaction solution is subjected to microwave treatment to obtain a xylan solution, wherein the microwave treatment is performed at a microwave frequency of 2450MHz, a treatment temperature from 40°C to 55°C for a microwaving time of 10 to 25min;

(4) enzymolysis: a xylanase is added to the xylan solution in step (3) for enzymolysis, the xylanase is added in an amount of 4 to 6 g/kg dry matter to obtain a crude xylooligosaccharide solution; wherein the enzymolysis is performed at a enzymolysis temperature from 50°C to 60°C for an enzymolysis time of 20 to 40 hours; the enzymolysis is a static reaction, and the xylanase used is a xylanase produced by *Trichoderma reesei* BLCY-007;

(5) enzyme inactivation: the crude xylooligosaccharide solution in step (4) is subjected to enzyme inactivation treatment, wherein the enzyme inactivation is a high-temperature enzyme inactivation, wherein the treatment is performed at an enzyme inactivation temperature from 85°C to 98°C for an enzyme inactivation time of 10 to 15 minutes;

(6) refining treatment: the solution after the enzyme inactivation in step (5) is subjected to decolorization treatment, ion exchange treatment, and concentration treatment to obtain an xylooligosaccharide solution; wherein activated carbon is used for decolorization during the decolorization treatment, and the activated carbon is added in an amount of 0.8% to 5% of the dry mass of the solution after the enzyme inactivation, the decolorization is performed at a temperature from 78°C to 85°C at a liquid flow rate of 20 to 30mL/min for a time of 15 to 30 minutes; an ion exchange column that is a combined column of cation exchange column-anion exchange column-cation exchange column is used in the ion exchange treatment, and the ion exchange treatment is performed at a temperature from 25°C to 35°C, with a flow rate of 15 to 25 mL/min;

the concentration treatment is performed by rotary concentration under vacuum at a working pressure of -0.1MPa and a working temperature from 60°C to 80°C, and the mass concentration of dry matter reaches 60% to 78% after the concentration treatment.

Beneficial effects:

1. The *Trichoderma reesei* BLCY-007 capable of producing xylanase at high yield is obtained for the first time in the present invention. The crude enzyme preparation extracted from the fungal fermentation broth of the strain has a xylanase activity of up to 508U/ml, which is more than 60% higher than that of the traditional *Trichoderma reesei,* thereby significantly reducing production costs.

2. The above crude enzyme preparation has an optimum working pH value of 5.5 to 6.5, which is beneficial for pollution control during production.

3. The xylanase produced by the *Trichoderma reesei* BLCY-007 of the present invention is an extracellular enzyme. The separation process is simple. The enzyme preparation can be obtained by simple centrifugation and washing, thereby reducing the production costs and decreasing energy consumption.

4. The crude enzyme preparation obtained based on the *Trichoderma reesei* BLCY-007 of the present invention does not contain unwanted enzymes such as cellulases. The crude enzyme preparation obtained from the *Trichoderma reesei* BLCY-007 has no filter paper enzyme activity (FPase) and has no carboxymethyl cellulase enzyme activity (CMCase).

5. The crude enzyme preparation obtained based on the *Trichoderma reesei* BLCY-007 does not contain toxins and can be safely used in the production of food raw material xylooligosaccharides.

6. The traditional production process requires acid treatment or alkali treatment for corncobs, and thus requires acid- resistant or alkali-resistant equipment, which requires a large one-time investment; and the preparation process thereof is complicated, not conducive to controlling the progress of reaction, has many side reactions that cause many by-products, has difficulty in product purification, and uses a large amount of acid and alkali in the process that causes a large amount of sewage discharge and serious environmental pollution. The present invention uses a high-temperature and high-pressure process to prepare xylooligosaccharides, which overcomes the shortcomings of the traditional production process. Traditional chemical treatment is replaced by a method in which no acid or alkali is used in the production process, a large amount of sewage discharge is avoided, and the pressure of environmental protection is reduced.

7. The high-yield xylanase-producing *Trichoderma reesei* BLCY-007 is used in the present invention, and the fungal fermentation broth thereof has an enzyme activity of up to 508U/ml, which is more than 60% higher than the enzyme activity of traditional xylanase, thereby significantly reducing production costs, and further increasing the yield of xylooligosaccharides at the same time.

8. The yield of xylan and the extraction rate of xylooligosaccharides of the present invention have been greatly improved compared with traditional production methods. The yield of xylan reaches higher than 64%, and the highest reaches 83%; the extraction rate of xylooligosaccharides is higher than 72%, and the highest reaches 87%; only adding xylanase once is needed for the enzymolysis in the process, which effectively reduces the production cost.

## Specific embodiments for Carrying Out the Invention

The embodiments of the present disclosure will be described in detail below in conjunction with examples. However, those skilled in the art will understand that the following examples are only used to illustrate the present disclosure, and not to limit the scope of the present disclosure. According to the following detailed description of the preferred embodiments, various objects and advantageous aspects of the present disclosure will become apparent to those skilled in the art.

### Example 1

A strain of *Trichoderma reesei* BLCY-007, which has an Accession number: CGMCC No. 17970, is provided. The number was obtained when the strain was deposited on June 14, 2019 in China General Microbiological Culture Collection Center, the Institute of Microbiology, Chinese Academy of Sciences, at the address: No.1, West Beichen Road, Chaoyang District, Beijing.

The mutagenesis and screening process of the aforementioned *Trichoderma reesei* BLCY-007 was as follows:

(1) Screening of original strain

Enrichment cultivation

The soil, which is near the xylooligosaccharides production workshop of Bailong Chuangyuan, Dezhou City, Shandong Province, was selected, and the top layer of the soil was removed with a small shovel; about 10g of the soil was taken from the ground at a depth of 10~20cm, diluted 10 times with sterile water, and added to a PDA medium (Potato Dextrose Agar) to for enrichment cultivation, and the cultivation was performed at a temperature from 24°C to 28°C for 36h.

The raw material components of the PDA culture medium were as follows: 1.0L of potato extract liquid, 20.0g of glucose, 15.0g of agar.

The potato extract liquid was prepared by a method as follows: 200 g of peeled potato was taken, cut into small pieces, added with 1.0L of water and boiled for 30min, filtered to remove potato pieces, and the filtrate was supplemented to 1.0L.

Separation of pure strain

A streaking method was used in this step. A large test tube containing 5ml of sterile water was taken, 2ml of the fungal solution obtained from the enrichment cultivation in step (1) was taken and added to the test tube and diluted,

shaken thoroughly for dispersion, a loop of the diluted solution was aseptically picked up by using an inoculation loop and subjected to the first parallel streaking of 3 to 4 streaks on one side of a plate medium of a petri dish; then the petri dish was turned about 60 degrees, and the remainder on the inoculation loop was burned off. After cooling, the second streaking was performed by the same method as that of the first streaking; and the third and fourth streakings were performed in sequence by the same method. After the end of streaking, the petri dish was covered with a lid, turned upside down, and incubated at 28°C to 38°C for 24 hours, then a single colony was picked up and inoculated on 10 slant culture medias to obtain slant seeds, numbered as 01 to 10, respectively.

The 01 to 10 slant seeds of were separately inoculated in shake flask culture medium and cultured at a temperature from 24°C to 28°C for 36 hours to obtain 01 to 10 shake flask fermentation broths. The xylanase enzyme activities of 01 to 10 shake flask fermentation broths were measured, and the 03 shake flask fermentation broth showed the highest enzyme activity, which is 105U/ml.

The raw material components of the plate medium were as follows: 1.0L of potato extract liquid, 20.0g of glucose, and 15.0g of agar.

Potato extract liquid: 200g of peeled potato was taken, cut into small pieces, added with 1.0L of water and boiled for 30min, filtered to remove potato pieces, and the filtrate was supplemented to 1.0L.

The components of the slant medium were as follows: 1.0 L of potato extract liquid, 20.0 g of glucose, and 15.0 g of agar.

The components of the shake flask culture medium were as follows: 200g of peeled potato, 20g of glucose, 3g of $KH_2PO_4$, 1.5g of $MgSO_4 \cdot 7H_2O$. After the above components were mixed, 1.0L of water was added and boiled for 30min, filtered to remove potato pieces, and the filtrate was supplemented to 1.0L.

(2) Mutation induced by mutagenic agent or ultraviolet radiation:

Mutagenesis screening

The strain in the fermentation broth of 03 shake flask was subjected to ultraviolet mutagenesis. The ultraviolet mutagenesis was performed by irradiating with a 15W ultraviolet lamp at a distance of 20 cm for a radiation time of 180 seconds, the obtained high-yield strain was then mutated with ethyl methyl sulfonate, and the finally obtained high-yield xylanase-producing strain was named as BLCY-007.

## Example 2

The method for culturing the *Trichoderma reesei* BLCY-007 described in Example 1 comprised the following steps:

(1) the *Trichoderma reesei* BLCY-007 was taken and inoculated in a PDA medium, an activating cultivation was performed for 12 hours at a temperature of 24°C to obtain an activated strain;

the PDA medium had raw material components as follows:

1.0L of potato extract liquid, 20.0g of glucose, 15.0g of agar;

the potato extract liquid was prepared by a method as follows: 200g of peeled potato was taken, cut into small pieces, added with 1.0L of water and boiled for 30min, filtered to remove potato pieces, and the filtrate was supplemented to 1.0L;

(2) the activated strain obtained in step (1) was taken and inoculated into a seed culture medium, a proliferating cultivation was performed for 24 hours at a temperature of 24°C to obtain a seed broth;

the seed culture medium had raw material components as follows:

200g of peeled potato, 20g of glucose, 3g of $KH_2PO_4$, 1.5g of $MgSO_4 \cdot 7H_2O$; the above components were mixed, then added with 1.0L of water and boiled for 30min, filtered to remove potato pieces, and the filtrate was supplemented to 1.0L;

(3) the seed broth prepared in step (2) was taken and inoculated in a fermentation medium at a volume percentage of 2%, and subjected to an expanding cultivation at a temperature of 24°C for 24 hours to obtain a fungal fermentation broth;

the fermentation medium had raw material components as follows, in terms of percentages by weight:

corncobs 25%, glucose 4%, beef extract 6%, peptone 1%, anhydrous magnesium sulfate 0.01%, dipotassium hydrogen phosphate 0.02%, ammonium sulfate 0.02%, balance of water, pH = 5.0 to 6.0.

**Example 3**

The method for culturing the *Trichoderma reesei* BLCY-007 described in Example 1 comprised the following steps:

(1) the *Trichoderma reesei* BLCY-007 was taken and inoculated in a PDA medium, an activating cultivation was performed for 24 hours at 28°C to obtain an activated strain;

the PDA medium had raw material components as follows:

1.0L of potato extract liquid, 20.0g of glucose, 15.0g of agar;

the potato extract liquid was prepared by a method as follows: 200g of peeled potato was taken, cut into small pieces, added with 1.0L of water and boiled for 30min, filtered to remove potato pieces, and the filtrate was supplemented to 1.0L;

(2) the activated strain obtained in step (1) was taken and inoculated into a seed culture medium, a proliferating cultivation was performed for 36 hours at 28°C to obtain a seed broth;

the seed culture medium had raw material components as follows:
200g of peeled potato, 20g of glucose, 3g of $KH_2PO_4$, 1.5g of $MgSO_4 \cdot 7H_2O$; the above components were mixed, then added with 1.0L of water and boiled for 30min, filtered to remove potato pieces, and the filtrate was supplemented to 1.0L;

(3) the seed broth prepared in step (2) was taken and inoculated in a fermentation medium at a volume percentage of 8%, and subjected to an expanding cultivation at 28°C for 36 hours to obtain a fungal fermentation broth;

the fermentation medium had raw material components as follows, in terms of percentages by weight:

corncobs 25%, glucose 4%, beef extract 6%, peptone 1%, anhydrous magnesium sulfate 0.01%, dipotassium hydrogen phosphate 0.02%, ammonium sulfate 0.02%, balance of water, pH = 5.0 to 6.0.

**Comparative Example 1**

The strain in 03 shake flask fermentation broth of Example 1 was used. Cultivation was performed by reference to the method of Example 2 to obtain a fungal fermentation broth.

**Comparative example 2**

The *Trichoderma reesei* purchased from Beijing Beina Chuanglian Biotechnology Research Institute was used as the culture strain. Cultivation was performed by reference to the method of Example 2 to obtain a fungal fermentation broth.

**Example 4**

Preparation of crude enzyme preparation: the fungal fermentation broths prepared in Example 2, Comparative Example 1 and Comparative Example 2 were subjected to centrifugation separation, and the centrifugation separation was performed under conditions of: crude enzyme preparation temperature: 4°C, centrifugal rotation speed: 10000 r/min, centrifugal time: 10 min. After centrifugation, supernatants were collected to obtain crude enzyme preparations.

**Experimental Example 1**

The crude enzyme preparations obtained in Example 4 were tested for their xylanase activities.

In the present disclosure, "enzyme activity" and "enzymatic activity" have the same meaning, and both refer to xylanase enzyme activity.

The determination of xylanase activity was performed according to "GBT 23874-2009, determination of xylanase activity in feed additives- spectrophotometric method". The determination method was briefly described as follows:

(i) Definition of xylanase activity unit
Under condition of 37°C and pH 5.5, an amount of enzyme required to release 1 μmol of reducing sugar per minute from a xylan solution with a concentration of 5 mg/ml was an enzyme activity unit, U.
(ii) Preparation of reaction enzyme solution of liquid sample
The above crude enzyme preparations were diluted with acetic acid-sodium acetate buffer solution (pH=5.5) to a certain volume, and the xylanase enzyme activity in the liquid sample after dilution was controlled between 0.04U/mL and 0.10U/mL.
(iii) Method for determination of enzyme activity
2ml of 100mg/ml xylan substrate (pH=5.5, acetic acid-sodium acetate buffer solution) was taken and added to a colorimetric tube, equilibrated at 37°C for 10min, then added with 2ml of the reaction enzyme solution of the liquid sample that had been equilibrated at 37°C. They were mixed well and incubated accurately at 37°C for 30 min. After the reaction was completed, 5ml of DNS reagent was added and mixed well to stop the reaction. Then the reaction mixture were boiled in a boiling water bath for 5 minutes, cooled to room temperature with tap water, added with distilled water to a certain volume of 25ml, mixed well, and tested for the absorbance $A_E$ at 540nm by using a standard blank as blank control.

Enzyme activity calculation formula:

$$X_D=[(A_E-A_B)\times K+C_0]\times 1000/(M\times t)$$

$$X=X_D\times N$$

wherein: X represents the xylanase activity of the liquid sample, U/ml; $X_D$ represents the xylanase activity of the reaction enzyme solution of the liquid sample, U/ml; $A_E$ represents the absorbance of the enzyme reaction solution; $A_B$ represents the absorbance of the enzyme blank solution; K represents the slope of the standard curve; Co represents the intercept of the standard curve; M represents the molar mass of xylose, 150.2g/mol; t represents the enzymolysis time, min; N represents the enzyme dilution multiple; 1000 represents the conversion factor, 1mmol=1000μmol.

The results were shown in Table 1:

Table 1

| No. | Example 2 BLCY-007 | Comparative Example 1 Original strain | Comparative Example 2 Beina Chuanglian Biotechnology Research Institute |
|---|---|---|---|
| Enzyme activity, U/mL | 506 | 101 | 163 |

It could be seen from Table 1 that compared with the original strain and other commercially available strain, the xylanase activity of the crude enzyme preparation obtained based on the *Trichoderma reesei* BLCY-007 had been greatly improved.

**Experimental Example 2**

A comparison experiment between the *Trichoderma reesei* strain disclosed in Chinese Patent CN1185336C and the BLCY-007 strain of present invention was performed.

The BLCY-007 strain of Example 1 was used as raw material. A liquid fermentation and a solid fermentation were performed respectively according to the methods described in step (3) and (4) of Examples, section 5 on pages 4 to 5 of the description of CN1185336C, and a liquid fermentation product-B and a solid fermentation product-B were obtained.

According to the xylanase enzyme activity determination method described in section 6 and the glucanase enzyme activity determination method described in section 7 on pages 5 to 8 of the description of CN1185336C, the

liquid fermentation product-B and the solid fermentation product-B were respectively tested for their xylanase enzyme activities and glucanase enzyme activities, and the results were as follows:

Table 2

| | | Enzyme activity of xylanase | Enzyme activity of glucanase |
|---|---|---|---|
| BLCY-007 strain | Liquid fermentation product-B | 587 U/ml | Without glucanase enzyme activity |
| | Solid fermentation product-B | 37605 U/g | Without glucanase enzyme activity |
| CN1185336C strain | Liquid fermentation product | 480 U/ml | 610 U/ml |
| | Solid fermentation product | 30000 U/g | 35000 U/g |

The methods for preparing xylooligosaccharides using corncobs as raw material are described below through specific examples.

The xylanase used in Example B1 was a crude enzyme preparation obtained based on *Trichoderma reesei* BLCY-007. The xylanase used in Comparative Examples B1 to B7 was xylanase *SP-min,* which was produced by Qingdao Viand Biological Co., Ltd.

In the cation exchange column used in the following examples, a strong acid cation resin, which was D001 macroporous cation exchange resin produced by Zhejiang Zhengguang Industrial Co., Ltd, was adopted.

In the anion exchange column used in the following examples, a weak base anion resin, which was D354FD macroporous weak base anion resin produced by Zhejiang Zhengguang Industrial Co., Ltd, was adopted.

The device used for microwave treatment in the following examples was MDS-6 microwave digestion/extraction instrument produced by Shanghai Sineo Microwave Chemistry Technology Co., Ltd., and its parameters were as follows: output power, 0 to 1000 W; temperature control range: 0°C to 250°C, temperature accuracy: ± 1°C; pressure control range: 0.1 to 5 MPa, pressure accuracy: 0.1 MPa.

Unless otherwise specified, "%" used in the following examples was a mass percentage.

In the following examples, the mass concentration referred to a mass concentration based on dry matter of corncobs.

In the following examples, the treatment pressure value should be understood as an incremental value based on 1 standard atmospheric pressure. For example, a treatment pressure of 0.1Mpa referred to an increase of 0. IMpa on the basis of 1 standard atmosphere.

**Example B1**

The method for preparing xylooligosaccharides by high-temperature and high-pressure treatment comprised the following steps:

(1) Pulping: 5g of corncobs (15% water content) was provided, crushed and the crushed product was sieved by a 100-mesh sieve, and the corncob powder that passed through the sieve was collected. The corncob powder was mixed with purified water to prepare a premix with a dry matter concentration of 9wt%, wherein the water used for the pulping was purified water.

(2) High-temperature and high-pressure treatment: the premix was placed in a pressure container and subjected to high-temperature and high-pressure treatment with a treatment temperature of 121°C and a treatment pressure of 0.10 MPa for a treatment time of 6 hours to obtain a crude extract liquid of xylan.

(3) Microwave treatment: the dry matter concentration of the crude extract liquid of xylan was adjusted to 4%, and the pH was adjusted to 4.7 to obtain 106ml of pre-reaction solution; the pre-reaction solution was placed in a microwave treatment device for microwave treatment to obtain a xylan solution; the microwave power of the microwave treatment device was 800W, the microwave frequency was 2450MHz, the treatment temperature was 50°C, and the microwaving time was 15min.

(4) Enzymolysis: a xylanase was added to the xylan solution and an enzymolysis was performed to obtain a crude xylooligosaccharide solution, in which the xylanase was added in an amount of 5g/kg dry matter; the enzymolysis

temperature was 52°C, the enzymolysis time was 24h, the pH value of the xylan solution was controlled at 5.5 to 6.5, and the enzymolysis was a static reaction.

The xylanase was a crude enzyme preparation obtained from the fermentation broth of *Trichoderma reesei* BLCY-007. The *Trichoderma reesei* BLCY-007 has an Accession number: CGMCC No. 17970, which was obtained when the strain was deposited on June 14, 2019 in China General Microbiological Culture Collection Center, the Institute of Microbiology, Chinese Academy of Sciences, at the address: No.1, West Beichen Road, Chaoyang District, Beijing.

(5) Enzyme inactivation: the above crude xylooligosaccharide solution was subjected to enzyme inactivation treatment. The enzyme inactivation temperature was 85°C, and the enzyme inactivation time was 10 minutes.

(6) Refining treatment: the product after enzyme inactivation was subjected to refining treatment, comprising:

decolorization, the product after enzyme inactivation passed through an activated carbon filter element, the activated carbon was added in an amount of 1% of dry basis, the temperature of the decolorization was 85°C, the time of the decolorization was 20min, and the liquid flow rate was 25mL/min during decolorization;

ion exchange treatment, the ion exchange column used herein was a combined column of cation exchange column-anion exchange column-cation exchange column, the temperature of the ion exchange treatment was 25°C, and the flow rate for the ion exchange treatment was 20 mL/min;

rotary concentration under vacuum, a rotary thin film vacuum concentration device was used for concentration treatment, in which the device had a working pressure of -0.1MPa, and a working temperature of 75 °C. The concentration was performed until the product had a dry matter concentration of 75%, thereby obtaining an xylooligosaccharide solution.

Theoretically, the mass of xylan obtained from 5g of corncobs was 1.8g. After testing, the mass of xylan in the xylan solution actually obtained in step (3) was 1.494g, and the mass of xylooligosaccharides obtained in step (6) was 1.299g. It can be calculated that the yield of xylan was 83%, and the extraction rate of xylooligosaccharides was 87%.

**Comparative Example B1**

Comparative Example B1 was similar to Example B1, except that: in step (2), the treatment temperature was 60° C, and the treatment pressure was 0.10 MPa. In step (4), the xylanase used was xylanase *SP-min* produced by Qingdao Viand Biotech INC, and the amount of xylanase added was 5 g/kg dry matter.
The test results showed that the yield of xylan and the extraction rate of xylooligosaccharides in this example were as follows: the yield of xylan was 31%, and the extraction rate of xylooligosaccharides was 38%.
The xylan in corncobs are a biological macromolecule, which in its natural state exists as a complex with other components such as cellulose and lignin. The three have a discontinuous laminate structure, which hinders the acid or alkali hydrolysis. Merely performing a high-pressure treatment could not destroy the discontinuous laminate structure, therefore, the yield of xylan and the extraction rate of xylooligosaccharides were not high.

**Comparative Example B2**

Comparative Example B2 was similar to Example B1, except that: in step (2), the treatment temperature was 121°C, and the treatment pressure was 0.01 MPa. In step (4), the xylanase *SP-min* produced by Qingdao Viand Biotech INC was used, and the amount of xylanase added was 5 g/kg dry matter.
The test results showed that the yield of xylan and the extraction rate of xylooligosaccharides in this example were as follows: the yield of xylan was 40%, and the extraction rate of xylooligosaccharides was 41%.
Similar to the reason for Comparative Example B1, merely performing a high-temperature treatment could not destroy the discontinuous laminate structure, therefore, the yield of xylan and the extraction rate of xylooligosaccharides were not high.

**Comparative Example B3**

Comparative Example B3 was similar to Example B1, except that: in step (2), the treatment temperature was 80°C, and the treatment pressure was 0.10 MPa. In step (4), the xylanase *SP-min* produced by Qingdao Viand Biotech INC was used, and the amount of xylanase added was 5 g/kg dry matter.

The test results showed that the yield of xylan and the extraction rate of xylooligosaccharides in this example were as follows: the yield of xylan was 44%, and the extraction rate of xylooligosaccharide was 42%.

Because the treatment temperature was too low to destroy the discontinuous laminate structure, the yield of xylan and the extraction rate of xylooligosaccharides were not high.

**Comparative Example B4**

Comparative Example B4 was similar to Example B1, except that: in step (2), the treatment temperature was 150°C, and the treatment pressure was 0.10 MPa. In step (4), the xylanase *SP-min* produced by Qingdao Viand Biotech INC was used, and the amount of xylanase added was 5 g/kg dry matter.

The test results showed that the yield of xylan and the extraction rate of xylooligosaccharides in this example were as follows: the yield of xylan was 36%, and the extraction rate of xylooligosaccharides was 35%.

Because the high-temperature treatment adopted an excessively high temperature, the xylan was excessively hydrolyzed to generate xylose, which reduced the yield of xylan and the extraction rate of xylooligosaccharides.

**Comparative Example B5**

Comparative Example B5 was similar to Example B1, except that: in step (2), the treatment temperature was 121°C, and the treatment pressure was 0.04 MPa. In step (4), the xylanase *SP-min* produced by Qingdao Viand Biotech INC was used, and the amount of xylanase added was 5 g/kg dry matter.

The test results showed that the yield of xylan and the extraction rate of xylooligosaccharides in this example were as follows: the yield of xylan was 42%, and the extraction rate of xylooligosaccharides was 41%.

Because the treatment pressure was too low to destroy the discontinuous laminate structure, the yield of xylan and the extraction rate of xylooligosaccharides were not high.

**Comparative Example B6**

Comparative Example B6 was similar to Example B1, except that: in step (2), the treatment temperature was 121°C, and the treatment pressure was 0.26 MPa. In step (4), the xylanase *SP-min* produced by Qingdao Viand Biotech INC was used, and the amount of xylanase added was 5 g/kg dry matter.

The test results showed that the yield of xylan and the extraction rate of xylooligosaccharide in this example were as follows: the yield of xylan was 38%, and the extraction rate of xylooligosaccharides was 36%.

Because the treatment pressure was too high, the xylan was excessively hydrolyzed to produce xylose, so that the yield of xylan and the extraction rate of xylooligosaccharides were reduced.

**Comparative Example B7**

Comparative Example B7 was similar to Example B1, except that:
in step (4), the xylanase *SP-min* produced by Qingdao Viand Biotech INC was used, and the amount of xylanase added was 5 g/kg dry matter.

Analysis and Test

In the above examples, the yield of xylan and the extraction rate of xylooligosaccharides were obtained by the following calculation formulas:

Formula 1: yield of xylan = detected mass of xylan / theoretically obtainable mass of xylan × 100%

Formula 2: extraction rate of xylooligosaccharides = mass of xylooligosaccharides/mass of xylan × 100%.

In the Formula 1, the mass of theoretically obtainable xylan = mass of corncobs × xylan content of corncob. Corncobs usually has a xylan content of 35% to 40%. The xylan content of the corncobs used in the above examples and comparative examples was 36%.

In the Formula 1, the method for detecting xylan content of the xylan solution was as follows: the xylan solution

was adjusted to a pH value of 5, added with 95% (by volume) ethanol in a volume four times of the that of the xylan solution, subjected to alcohol precipitation overnight and then centrifuged at 3000r/min for 10 minutes, and the precipitate was collected, added with a certain amount of 7% $H_2SO_4$ for hydrolysis at 100°C for 2 hours, then neutralized, supplemented to a certain volume, filtered, tested for the mass of reducing sugar in the filtrate, then the mass was multiplied by the xylan polymerization factor 0.9 to give the mass of xylan. DNS method was used for determination of reducing sugar.

In the Formula 1, the detected mass of xylan = 0.9 × mass of reducing sugar determined by DNS method.

In the Formula 2, the method for detection of xylooligosaccharides was in accordance with GB/T 35545-2017 (Appendix A: High performance liquid chromatography) for detection of xylooligosaccharides.

The parameters of some steps and the product yields of Example B1 and Comparative Examples B1 to B7 were shown in Table 3 below:

Table 3

| | Step (2) | | Step (4) | Yield | |
|---|---|---|---|---|---|
| | Treatment Temperature (°C) | Treatment Pressure (MPa) | Enzyme | Yield of xylan (%) | Yield of xylooligosaccharides (%) |
| Example B1 | 121 | 0.10 | Crude enzyme preparation of BLCY-007 | 83 | 87 |
| Comparative Example B1 | 60 | 0.10 | *SP-min* Qingdao Viand Biotech INC | 31 | 38 |
| Comparative Example B2 | 121 | 0.01 | | 40 | 41 |
| Comparative Example B3 | 80 | 0.10 | | 44 | 42 |
| Comparative Example B4 | 150 | 0.1 | | 36 | 35 |
| Comparative Example B5 | 121 | 0.04 | | 42 | 41 |
| Comparative Example B6 | 121 | 0.26 | | 38 | 36 |
| Comparative Example B7 | 121 | 0.10 | | 61 | 64 |

It can be seen from the experimental results in Table 3 that the preparation of xylooligosaccharides using corncobs as raw material in Example B1 had a significantly improved yield of xylooligosaccharides, which indicated that unexpected technical effects were obtained.

Although the specific embodiments of the present disclosure have been described in detail, those skilled in the art will understand that various modifications and changes can be made to the details according to all the teachings that have been disclosed, and these changes are within the protection scope of the present disclosure. The full scope of the present disclosure is given by the appended claims and any equivalents thereof.

## Claims

1. A strain of *Trichoderma reesei* BLCY-007, which has an accession number: CGMCC No. 17970, wherein said accession number was obtained when the strain was deposited on June 14, 2019 in China General Microbiological Culture Collection Center (CGMCC).

2. A method for culturing the *Trichoderma reesei* BLCY-007 according to claim 1, comprising the following steps:

   (i) inoculating the *Trichoderma reesei* BLCY-007 into a PDA medium, performing an activating cultivation under a temperature from 24°C to 28°C for 12 to 24 hours to obtain an activated strain;
   (ii) inoculating the activated strain obtained in step (i) into a seed culture medium, and performing a proliferating

cultivation under a temperature from 24°C to 28°C for 24 to 36 hours to obtain a seed broth;
(iii) inoculating the seed broth obtained in step (ii) into a fermentation medium at a volume percentage of 1% to 10%, and performing an expanding cultivation at a temperature from 24°C to 28°C for 24 to 36 hours to obtain a fungal fermentation broth.

**3.** The method according to claim 2, **characterized in that**, said seed culture medium in step (ii) has raw material components as follows:
200g of peeled potato, 20g of glucose, 3g of $KH_2PO_4$, 1.5g of $MgSO_4 \cdot 7H_2O$; the above components are mixed, added with 1.0L of water and boiled for 30min, filtered to remove potato pieces, and the filtrate is supplemented to 1.0L.

**4.** The method according to claim 2, **characterized in that**, said fermentation medium in step (iii) has raw material components as follows, in terms of weight percentages:
corncobs 25%, glucose 4%, beef extract 6%, peptone 1%, anhydrous magnesium sulfate 0.01%, dipotassium hydrogen phosphate 0.02%, ammonium sulfate 0.02%, balance of water, pH = 5.0 to 6.0.

**5.** The method according to claim 2, **characterized in that** the PDA medium in step (i) has raw material components as follows:

1.0L of potato extract liquid, 20.0g of glucose, 15.0g of agar;
the potato extract liquid is prepared by the following method: 200 g of peeled potato is taken, cut into small pieces, added with 1.0L of water and boiled for 30min, filtered to remove potato pieces, and the filtrate is supplemented to 1.0L.

**6.** Use of the *Trichoderma reesei* BLCY-007 according to claim 1 in the production of xylanase.

**7.** The use according to claim 6, **characterized in that** the use comprises steps as follows:
providing the fungal fermentation broth obtained by the method according to claim 2, subjecting it to centrifugation separation, washing the obtained fungal cells, performing a second centrifugation, and retaining the precipitate as the crude xylanase enzyme preparation.

**8.** The use according to claim 6, **characterized in that** the use comprises steps are as follows:

(1) providing the fungal fermentation broth obtained by the method according to claim 2;
(2) subjecting the fungal fermentation broth to centrifugation separation;
(3) collecting a supernatant from the product of the previous step, wherein the supernatant is the crude xylanase enzyme preparation.

**9.** The use according to claim 7 or 8, wherein the centrifugation separation is performed at 4°C and 10000 r/min for 10 min.

**10.** Use of the *Trichoderma reesei* BLCY-007 according to claim 1 in the production of xylooligosaccharides.

**11.** A method for preparing a crude enzyme preparation, comprising:

(1) providing the fungal fermentation broth obtained by the method according to claim 2;
(2) subjecting the fungal fermentation broth to centrifugation separation;
(3) collecting a supernatant from the product of the previous step, wherein the supernatant is the crude xylanase enzyme preparation.

**12.** A method for preparing xylooligosaccharides, comprising:

(1) preparing a crude xylanase enzyme preparation by the method according to claim 11;
(2) subjecting a xylan to enzymolysis treatment by using said crude xylanase enzyme preparation to obtain xylanoligosaccharides;
preferably, the enzymolysis treatment is performed at a temperature from 50°C to 60°C; preferably, the enzymolysis treatment is performed at a pH of 5.5 to 6.5.

**13.** A method for preparing xylooligosaccharides, comprising the following steps:

preparing a premix by crushing and sieving corncobs and adding water thereto;
subjecting the premix to high-temperature and high-pressure treatment to obtain a crude extract liquid of xylan, wherein the treatment is performed at a treatment temperature from 95°C to 140°C and a treatment pressure of 0.05 to 0.25 MPa;
adjusting the crude extract liquid of xylan to a mass concentration of 4% to 6% and to a pH between 4.2 and 4.8, and performing a microwave treatment to obtain a xylan solution, wherein the microwave treatment is performed at a microwave frequency of 2450 MHz and a treatment temperature from 40°C to 55°C for a microwaving time of 10 to 25 minutes;
adding a xylanase into the xylan solution to perform enzymolysis to obtain a crude xylooligosaccharide solution; wherein the xylanase used is a xylanase produced by the following strain: said strain is *Trichoderma reesei* BLCY-007, which has an Accession number: CGMCC No. 17970, which was obtained when the strain was deposited on June 14, 2019 in China General Microbiological Culture Collection Center (CGMCC);
subjecting the crude xylooligosaccharide solution to enzyme inactivation treatment, decolorization treatment, ion exchange treatment, and concentration treatment to obtain an xylooligosaccharide solution.

**14.** The method for preparing xylooligosaccharides according to claim 13, **characterized in that** the corncobs are crushed to a particle size that is capable of passing through 80 to 120 mesh sieve, and the premix has a mass concentration of 8% to 12%.

**15.** The method for preparing xylooligosaccharides according to claim 13, **characterized in that** the high-temperature and high-pressure treatment is performed at a treatment temperature from 115°C to 128°C and a treatment pressure of 0.09 to 0.18 MPa for a treatment time of 4 to 8 hours.

**16.** The method for preparing xylooligosaccharides according to claim 13, **characterized in that** the xylan solution is adjusted to a mass concentration of 4% to 6% before enzymolysis, wherein the xylanase is added in an amount of 4 to 6 g/kg dry matter.

**17.** The method for preparing xylooligosaccharides according to claim 13, **characterized in that** the enzymolysis is performed at an enzymolysis temperature from 50°C to 60°C for an enzymolysis time from 20 to 40 hours.

**18.** The method for preparing xylooligosaccharides according to claim 13, **characterized in that** the enzyme inactivation treatment is performed at an enzyme inactivation temperature from 85°C to 98°C for an enzyme inactivation time from 10 to 15 minutes.

**19.** The method for preparing xylooligosaccharides according to claim 13, **characterized in that** the decolorization treatment is performed by using activated carbon, the activated carbon is added in an amount of 0.8% to 5% of the dry mass of the crude xylooligosaccharide solution, the decolorization is performed at a temperature from 78°C to 85°C, at a liquid flow rate of 20 to 30 mL/min for a time from 15 to 30 minutes.

**20.** The method for preparing xylooligosaccharides according to claim 13, **characterized in that** the ion exchange treatment is performed on an ion exchange column that is a combined column of cation exchange column-anion exchange column-cation exchange column, at a temperature from 25°C to 35°C and at a flow rate of 15 to 25 mL/min.

**21.** The method for preparing xylooligosaccharides according to claim 13, **characterized in that** the concentration treatment is performed by vacuum rotary concentration at a working pressure of -0.1MPa, a working temperature from 60°C to 80°C, and the concentration of dry matter in the crude xylooligosaccharide solution is 60% to 78% after the concentration treatment.

**22.** The method for preparing xylooligosaccharides according to claim 13, wherein the xylanase used for the enzymolysis is a crude enzyme preparation isolated from the fermentation product of *Trichoderma reesei* BLCY-007.

**23.** The method for preparing xylooligosaccharides according to claim 22, wherein the crude enzyme preparation is prepared by a method comprising:

(1) providing the fungal fermentation broth obtained;
(2) subjecting the fungal fermentation broth to centrifugation separation;
(3) collecting a supernatant from the product of the previous step, wherein the supernatant is the crude xylanase enzyme preparation.

**24.** The method for preparing xylooligosaccharides according to claim 23, wherein the method for preparing the fermentation broth of *Trichoderma reesei* BLCY-007 in step (1) comprises:

(i) inoculating the *Trichoderma reesei* BLCY-007 into a PDA medium, performing an activating cultivation under a temperature from 24°C to 28°C for 12 to 24 hours to obtain an activated strain;
(ii) inoculating the activated strain obtained in step (i) into a seed culture medium, and performing a proliferating cultivation under a temperature from 24°C to 28°C for 24 to 36 hours to obtain a seed broth;
(iii) inoculating the seed broth obtained in step (ii) into a fermentation medium at a volume percentage of 1% to 10%, and performing an expanding cultivation at a temperature from 24°C to 28°C for 24 to 36 hours to obtain a fungal fermentation broth.

**25.** The method for preparing xylooligosaccharides according to claim 24, wherein the PDA medium in step (i) has raw material components as follows:
1.0L of potato extract liquid, 20.0g of glucose, 15.0g of agar.

**26.** The method for preparing xylooligosaccharides according to claim 24, wherein the seed culture medium in step (ii) has raw material components as follows:
200g of peeled potato, 20g of glucose, 3g of $KH_2PO_4$, 1.5g of $MgSO_4 \cdot 7H_2O$; the above components are mixed, added with 1.0L of water and boiled for 30min, filtered to remove potato pieces, and the filtrate is supplemented to 1.0L.

**27.** The method for preparing xylooligosaccharides according to claim 24, wherein the fermentation medium in step (iii) has raw material components as follows, in terms of weight percentages:
corncobs 25%, glucose 4%, beef extract 6%, peptone 1%, anhydrous magnesium sulfate 0.01%, dipotassium hydrogen phosphate 0.02%, ammonium sulfate 0.02%, balance of water, pH = 5.0 to 6.0.

**INTERNATIONAL SEARCH REPORT**

International application No.
**PCT/CN2020/100482**

**A. CLASSIFICATION OF SUBJECT MATTER**

C12N 1/14(2006.01)i; C12N 9/24(2006.01)i; C12P 19/14(2006.01)i; C12P 19/00(2006.01)i; C12P 19/12(2006.01)i; C12P 19/04(2006.01)i

According to International Patent Classification (IPC) or to both national classification and IPC

**B. FIELDS SEARCHED**

Minimum documentation searched (classification system followed by classification symbols)

C12N; C12P

Documentation searched other than minimum documentation to the extent that such documents are included in the fields searched

Electronic data base consulted during the international search (name of data base and, where practicable, search terms used)

CNABS, CPRSABS, CNTXT, VEN, WPI, EPODOC, CNKI, EPTXT, WOTXT, USTXT, Web of Knowledge, 超星科技数字图书馆, 万方数据知识服务平台, CNKI: 山东百龙创园生物科技股份有限公司, 窦宝德, 李方华, 刘伟, 窦光朋, 干昭波, 邵先豹, 里氏木霉, Trichoderma reesei, BLCY-007, CGMCC No.17970, 木聚糖酶, xylanase, 木聚糖内切酶, xyn, XynI, XynII, XynIII, XYL-I, XYL-II, 和XYL-III, 低聚糖, 寡糖, oligosaccharides, 低聚木糖, 木寡糖

**C. DOCUMENTS CONSIDERED TO BE RELEVANT**

| Category* | Citation of document, with indication, where appropriate, of the relevant passages | Relevant to claim No. |
|---|---|---|
| PX | CN 110564629 A (SHANDONG BAILONG CHUANGYUAN BIO-TECH CO., LTD.) 13 December 2019 (2019-12-13)<br>claims 1-10 | 1-7, 9-10 |
| PX | CN 110628846 A (SHANDONG BAILONG CHUANGYUAN BIO-TECH CO., LTD.) 31 December 2019 (2019-12-31)<br>claims 1-8 | 1, 6, 10, 13-22 |
| PX | CN 109988715 A (VLAND BIOTECH GROUP CO., LTD. et al.) 09 July 2019 (2019-07-09)<br>claims 1-4, description pages 2-4 | 1-6, 8-12 |
| X | CN 1385519 A (ZHEJIANG UNIVERSITY) 18 December 2002 (2002-12-18)<br>claims 1-8, description pages 2-5 | 1-12 |
| Y | CN 1385519 A (ZHEJIANG UNIVERSITY) 18 December 2002 (2002-12-18)<br>claims 1-8, description pages 2-5 | 13-27 |
| Y | CN 104774887 A (XUCHANG UNIVERSITY) 15 July 2015 (2015-07-15)<br>claim 1, description paragraph 23 | 13-27 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 September 2020** | **15 October 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/100482**

**C. DOCUMENTS CONSIDERED TO BE RELEVANT**

| Category* | Citation of document, with indication, where appropriate, of the relevant passages | Relevant to claim No. |
|---|---|---|
| Y | CN 104762419 A (SOUTH CHINA UNIVERSITY OF TECHNOLOGY) 08 July 2015 (2015-07-08)<br>embodiment 1, description paragraphs 19-22 | 13-27 |
| A | CN 104130950 A (SHANDONG BAILONG CHUANGYUAN BIOTECHNOLOGY CO., LTD.) 05 November 2014 (2014-11-05)<br>entire document | 1-27 |
| A | CN 106148243 A (SHANDONG BAILONG CHUANGYUAN BIOTECHNOLOGY CO., LTD.) 23 November 2016 (2016-11-23)<br>entire document | 1-27 |
| A | CN 106434494 A (SHANDONG BAILONG CHUANGYUAN BIO-TECH CO., LTD.) 22 February 2017 (2017-02-22)<br>entire document | 1-27 |
| A | US 2014127754 A1 (DYADIC INTERNATIONAL, INC.) 08 May 2014 (2014-05-08)<br>entire document | 1-27 |
| A | EP 2336152 A1 (SUED-CHEMIE AG) 22 June 2011 (2011-06-22)<br>entire document | 1-27 |
| A | JP 2010124720 A (OJI PAPER CO LTD) 10 June 2010 (2010-06-10)<br>entire document | 1-27 |

Form PCT/ISA/210 (second sheet) (January 2015)

**INTERNATIONAL SEARCH REPORT**
**Information on patent family members**

International application No.
**PCT/CN2020/100482**

| Patent document cited in search report | Publication date (day/month/year) | Patent family member(s) | Publication date (day/month/year) |
|---|---|---|---|
| CN 110564629 A | 13 December 2019 | None | |
| CN 110628846 A | 31 December 2019 | CN 110628846 B | 03 July 2020 |
| CN 109988715 A | 09 July 2019 | None | |
| CN 1385519 A | 18 December 2002 | CN 1185336 C | 19 January 2005 |
| CN 104774887 A | 15 July 2015 | None | |
| CN 104762419 A | 08 July 2015 | None | |
| CN 104130950 A | 05 November 2014 | CN 104130950 B | 25 January 2017 |
| CN 106148243 A | 23 November 2016 | CN 106148243 B | 19 March 2019 |
| CN 106434494 A | 22 February 2017 | EP 3550011 A1 | 09 October 2019 |
| | | WO 2018099366 A1 | 07 June 2018 |
| | | CN 106434494 B | 07 November 2017 |
| | | US 2019284521 A1 | 19 September 2019 |
| | | CA 3045528 A1 | 07 June 2018 |
| US 2014127754 A1 | 08 May 2014 | None | |
| EP 2336152 A1 | 22 June 2011 | CA 2785311 C | 10 January 2017 |
| | | EP 2516451 A2 | 31 October 2012 |
| | | US 8679795 B2 | 25 March 2014 |
| | | US 2013109062 A1 | 02 May 2013 |
| | | BR 112012015695 A2 | 08 May 2018 |
| | | EP 2336152 B1 | 13 August 2014 |
| | | WO 2011080078 A2 | 07 July 2011 |
| | | ES 2523242 T3 | 24 November 2014 |
| | | WO 2011080078 A3 | 13 October 2011 |
| | | CA 2785311 A1 | 07 July 2011 |
| JP 2010124720 A | 10 June 2010 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201910597371
- CN 201910596633
- CN 201910996538
- CN 201910997521
- CN 105154412 A
- IN 201741043810 A
- CN 1185336 C